# EUROPEAN PATENT APPLICATION

(11) **EP 0 875 227 A1**
(43) Date of publication of application: **04.11.1998**
(21) Application number: 97107095.8
(22) Date of filing: 29.04.1997
(51) Int. Cl.: A61F 13/56, A44B 18/00, A61F 13/62

(54) **Adhesive-free female fastening portion for a mechanical fastening device**

(71) Applicant: THE PROCTER & GAMBLE COMPANY, Cincinnati, Ohio 45202 (US)
(72) Inventor: Müller, Jörg, 61184 Karben (DE); Busam, Ludwig, 65510 Hünstetten (DE)
(74) Representative: Kohol, Sonia

(57) **Abstract**

A female fastening portion (30) for a refastenable mechanical fastening device (54) is described, said female fastening portion (30) being capable of engaging a complementary male fastening portion (84), said female fastening portion (30) comprising a textile material (32), said textile material (32) being disposed adjacent a substrate (34), whereby said textile material (32) is non-adhesively bonded to said substrate (34), characterised in that said textile material (32) has a basis weight not greater than 60 grammes per square metre. In a further aspect of the invention, the female fastening portion (30) is applied to a disposable absorbent article (50).

## Description

### Field of the invention

The present invention relates to an adhesive-free female fastening portion for a refastenable mechanical fastening device. Its application is intended for use on disposable absorbent articles.

### Background of the invention

Disposable absorbent articles, in particular, disposable diapers are well-known articles of manufacture that are designed to be worn principally by infants and incontinence sufferers. Such diapers are worn about the lower torso of the wearer and are intended to absorb and contain urine and other bodily discharges, thus preventing the soiling, wetting, or similar contamination of articles (for example, clothing, bedding, other persons, etc.) that may come into contact with such a diaper in use.

When using a disposable diaper, the diaper user fits the diaper on the wearer and fastens it about the waist of the wearer with the aid of a fastening device to effect a side closure. Fitting the diaper about the wearer usually requires the front and the back waist portions of the diaper to overlap each other. Since proper and sustained fit about the waist and legs of the wearer is vital for optimal performance in terms of minimising the leakage of body exudates out of the diaper, a diaper fastening device must be capable of providing an effective side closure in which the front and back waist portions are maintained in an overlapping configuration. As the diaper is worn, the overlapping portions are subjected to forces that tend to cause the portions to shift position relative to each other. Unless such shifting is limited, the fit and containment characteristics of the diaper deteriorate as the diaper is worn. Thus, the fastening device must be designed to securely engage so it does not separate due to the peel forces and shear stresses encountered by the fastening device during use.

The use of fastening devices for securing the front and back portions on a disposable absorbent article is widely known. Examples of adhesive tape tab fastening devices are disclosed in US RE 26,151, US 3,848, 594, US 4,985,025, EP 0 286 030 and many other prior art documents. While tape tab fastening devices provide a secure means for fixing a disposable absorbent article around the waist of the wearer during use, they are not completely satisfactory. Such fastening devices are often only usable upon initial fixation of the disposable absorbent article and refastenability is not a concomitant benefit. Furthermore, the fastening devices display an unsatisfactory resistance to contamination from oils and powders. Thus, the prior art documents disclose many alternatives as potential solutions to such deficiencies. For example mechanical fastening devices have been contemplated and are disclosed in many publications such as US 3,110,3112, US 4,259,957 and EP 0 235 014. Such mechanical fastening devices exhibit a reduced sensitivity to contamination and have the advantage of refastenability. As a consequence, these mechanical fastening devices have gained in popularity over the last few years. These mechanical fastening devices generally constitute two elements: a female fastening portion comprising a loop material and a male fastening portion comprising a hook fastening material.

After establishing the principal benefits of the mechanical fastening devices over adhesive tape tab fastening systems, it was discovered that a problem arose in connection with adhesive-based female fastening portions. Typically, the female fastening portion comprises a textile material. It was recognised that the adhesive attachment of the textile material to a base substrate affected the functional performance of the loops. Generally, the adhesive tended to bleed through the textile material structure resulting in the loops sticking to the textile material and rendering a representative proportion of the loops completely ineffectual for engagement with the hook fastening material of the male fastening portion.

In order to address such a problem, US 5,554,239 focuses on the use of a lower basis weight in the first zone of the adhesive to prevent bleeding from the first zone to the loop material. Nevertheless, the use of a higher basis weight in the peripheral portions of the loop material in the second zone of the adhesive results in bleeding and consequently, deadening of the loop material. Therefore, the functional performance of the loop material for engagement is greatly impaired and in some cases destroyed.

In contrast, European patent application number 9606398, filed on 23 May 1996, describes a preferred embodiment in which the assembly for use as a female fastening portion on a disposable absorbent article comprises a first layer and a second layer, affixed to each other by means of thermobonding, and loop elements, which are attached to the second layer. The first layer is dense and the second layer is formed by interlacing the yarns in a warp or weft pattern. US 5,256,231 describes a sheet of loop material that is designed to be cut into pieces to form loop portions for mechanical fastening devices. The sheet of loop material is made by forming a sheet of longitudinally oriented polymeric fibres so that the sheet has arcuate portions projecting in the same direction from spaced anchor portions, and then by forming at least a portion of a backing around the spaced anchor portions by extruding thermoplastic material onto the anchor portions. The non-woven fibre sheet has a basis weight in the range of 5 to 300 grammes per square metre and preferably in the range from 15 to 100 grammes per square metre for applications in which the fastening device will be opened less than ten times, and in the range from 75 to 150 grammes per square metre for applications in which the fastening device will be opened more than ten times. In a further embodiment, yarns may be used with a denier ranging from 50 to 300.

As a result of the above prior art attempts, it has been recognised by those skilled in the art that it would be desirable to provide a female fastening portion with an extremely high number of functionally operating loops. Furthermore, it would be most advantageous to reduce the basis weight of the textile material from a functional and aesthetic point of view. The basis weight reduction results in better material utilisation, allows the artwork on the substrate underlying the textile material to be more clearly highlighted and leads to an improvement in tactile qualities. It is also known that using current technologies, it is difficult to reduce the basis weight of the female fastening portion without causing a degradation in the performance of the mechanical fastening device, i.e., the shear stress and peel force values deteriorate.

It has now been discovered that the benefits of the present invention range from a female fastening portion exhibiting a superior functional performance when engaged with the hook fastening material of a male fastening portion; to a low basis weight assembly, which is most attractive from a materials' utilisation aspect; to a female fastening portion with a very satisfactory peel force resistance value when engaged with the hook fastening material of the male fastening portion; to a softer and more gentle female fastening portion; to a fastening device that is adhesive free; and to a mechanical fastening device that leads to a high level of user satisfaction and confidence.

### Summary of the invention

The female fastening portion of the present invention represents a constituent member of a mechanical fastening device, the other constituent member being a male fastening portion, which is designed to mechanically engage with the female fastening portion. In particular, hook type male fastening portions engage with loop type female fastening portions.

In one aspect, the present invention concerns a female fastening portion for a refastenable mechanical fastening device wherein the female fastening portion is capable of engaging a complementary male fastening portion. The female fastening portion comprises a textile material, which is disposed adjacent to a substrate. The textile material is non-adhesively bonded to the substrate and has a basis weight that is not greater than 60 grammes per square metre. More particularly, the textile material of the female fastening portion comprises a basis weight not greater than 40 grammes per square metre, preferably not greater than 34 grammes per square metre and more preferably not greater than 28 grammes per square metre. The female fastening portion may either be thermobonded or extrusion bonded to the substrate. In a preferred embodiment of the present invention, the textile material has a three bar warp pattern comprising loops, courses and wales. In another preferred embodiment of the present invention, the textile material has a two bar warp pattern comprising only courses and wales.

In another aspect of the present invention, the female fastening portion is applied to a disposable absorbent article, such as a diaper. The substrate, to which the textile material is attached, can comprise either a film adjacent to the outside surface of the body portion with a layer of adhesive interposed between or just simply the outside surface of the body portion.

### Brief description of the drawings

It is believed that the invention will be better understood from the foregoing description in conjunction with the accompanying drawings in which:
Figure 1 is an enlarged sectional view of the female fastening portion according to the teachings of the present invention;
Figure 2 shows a plan view photograph of a three bar warp knitted female fastening portion;
Figure 3 illustrates the construction of the three bar warp knitted female fastening portion as shown in Figure 2; and
Figure 4 details a partially cut-away perspective view of a diaper embodying the refastenable mechanical fastening device of the present invention.

### Detailed description of the invention

As used herein, the term "textile" includes knit material, woven material, and scrim. As used herein, the term "disposable absorbent article" refers to articles which absorb and contain body exudates; and more specifically, refers to articles which are placed against or in proximity to the body of the wearer to absorb and contain the various exudates discharged from the body and which are intended to be discarded after a single use (i.e., they are not intended to be laundered or otherwise restored or reused) and, preferably, to be recycled, composted or otherwise disposed of in an environmentally compatible manner. As used herein, the term "diaper" refers to a garment generally worn by infants or incontinence sufferers that is drawn up between the legs and fastened about the waist of the wearer. It should be understood, however, that the present invention is also applicable to other disposable absorbent articles such as diapers, training pants, incontinence briefs, feminine hygiene garments or panties, bibs, hospital gowns, caps, bandages and the like. As used herein, the term "shear stress" refers to the distributed forces acting tangentially to the surface of contact of the members of the fastening device (or along the x/y plane). During the wearing of a disposable absorbent article, shear stress tends to cause the members of the fastening device to shift with respect to each other. Shear stress is to be distinguished from "peel forces" which act on the fastening portions of the fastening device so as to separate and disengage from each other in the z-direction. A disposable absorbent article is typically subjected to peel forces in at least three ways. Peel forces are generated by the movements of the wearer during use as they tend to cause the first and second fastening portions of the fastening device to pull away from each other, by the wearer in trying to unfasten the fastening device during wear and by the user to check the disposable absorbent article for soiling or to remove the disposable absorbent article from the wearer. The fastening device is preferably designed to have a resistance to peel forces (peel force resistance) with respect to only the movement and wearer generated methods. Therefore, the peel force resistance should only be great enough to prevent failure of the fastening device during the first two methods, but low enough to allow the user to check the disposable absorbent article for soiling or to remove the disposable absorbent article from the wearer without undue difficulty or tearing of other members of the disposable absorbent article. It is desirable to design a fastening device capable of resisting shear stresses and peel forces generated by the wearer, but having a peel force resistance low enough to allow the user to easily remove the disposable absorbent article or check the disposable absorbent article for soiling.

Refastenable mechanical fastening devices of the present invention have been found to be particularly useful and beneficial when applied to disposable absorbent articles. The refastenable mechanical fastening device of the present invention comprises a female fastening portion, which is capable of engaging a complementary male fastening portion.

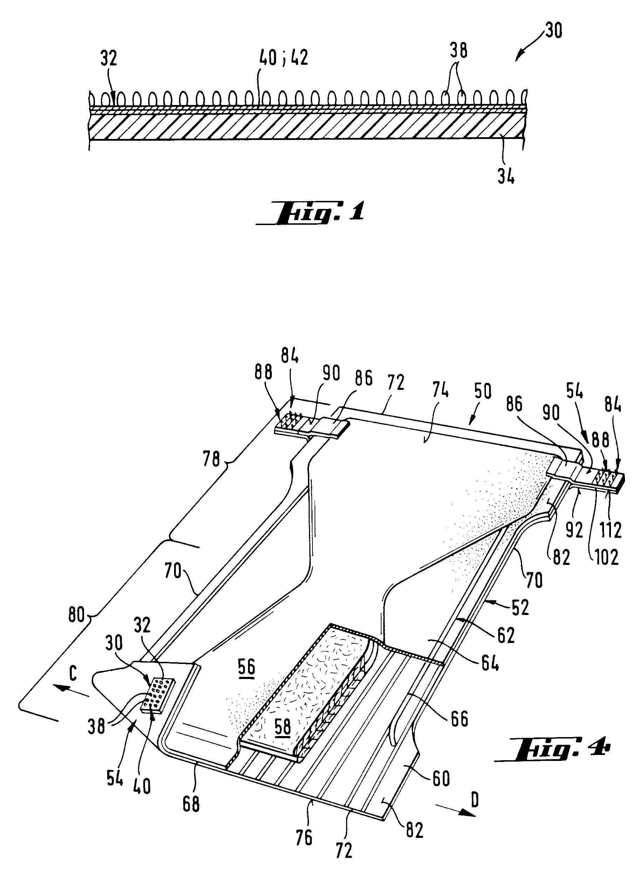

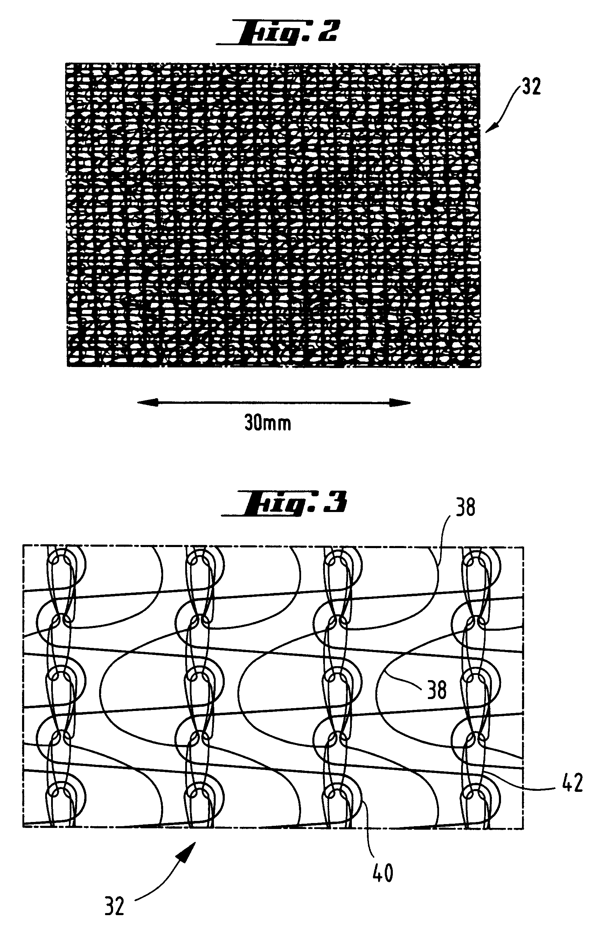

In one aspect of the present invention, a female fastening portion for a refastenable mechanical fastening device is described. Figures 1 and 2 show a female fastening portion **30** comprising a textile material **32**, which is disposed adjacent to a substrate **34** whereby the textile is non-adhesively bonded to the substrate **34**. The basis weight of the textile material **32** is not greater than 60 grammes per square metre. More specifically, the basis weight of the textile **32** is not greater than 40 grammes per square metre, preferably not greater than 34 grammes per square metre and more preferably not greater than 28 grammes per square metre.

The female fastening portion **30** comprises a loop type fastening material consisting of a plurality of loops **38.** The female fastening portion **30** is a flexible textile material **32** that may comprise any suitable yarn material. The yarn materials include, but are not limited to, polypropylene, polyethylene terephthalate, polyamide or other like synthetic or natural fibres. In a preferred embodiment of the present invention, as is seen from Figures 2 and 3, the female fastening portion **30** is formed of a textile material **32** having a three bar warp knit construction comprising loops **38**, courses **40** (yarns in the machine direction) and wales **42** (yarns in the cross machine direction). The loops **38**, courses **40** and wales **42** are preferably of polyamide. The courses **40** range from 5 to 25 courses **40** per centimetre, the wales **42** range from 3 to 12 wales **42** per centimetre and the loops **38** range from 10 to 200 loops **38** per square centimetre. In a more preferred embodiment of the present invention, the textile material **32** comprises 15 courses **40** per centimetre, 5 wales **42** per centimetre and 40 loops **38** per square centimetre. The loops **38** of the textile material **32** may be formed with a loop height ranging from about 0.2 to 20 millimetres, a loop denier ranging from 8 to 78 and a basis weight, which is not greater than 60 grammes per square metre. Typically, the basis weight is not greater than 40 grammes per square metre, preferably not greater than 34 grammes per square metre and more preferably, not greater than 28 grammes per square metre. The loops **38** can be stabilised through napping, thermosetting or the like.

In another embodiment of the present invention, the female fastening portion **30** comprises a two bar warp knit construction comprising courses **40** ranging from 5 to 25 courses **40** per centimetre, preferably 15 per centimetre, and wales **42** ranging from 3 to 12 wales **42** per centimetre, preferably 5 per centimetre.

In particular, the yarns for the loops **38**, courses **40** and wales **42** are composed of yarn having about 1 to 30 individual filaments, with preferably 30 filaments of yarn for the loops **38**, 1 filament for the courses **40** and 1 filament for the wales **42**. The yarns typically have a denier in the range from 8 to 78 denier. In a preferred embodiment of the present invention, the denier value for the loops **38** is 44 denier, and for the courses **40** and wales **42** is 22 denier, respectively.

The loops **38** are preferably constructed in such a way that the shear stresses and the peel forces show an equivalent distribution in both directions **C** and **D**, as is evident from Figure 4 (for example, stretch direction of elastic members **66**). The preferred pattern involves the loops **38** being oriented either all in the same direction or half of the loops **38** being oriented in one direction and the other half being oriented in the other direction in an alternating fashion.

The textile material **32** is either thermobonded or extrusion bonded to the substrate **34** using conventional means known to the man skilled in the art. Thermobonding uses heat to bond or stabilise the textile material **32** to the substrate **34**. Typically, the substrate **34** comprises thermoplastic fibres. All or part of the fibres act as thermal binders, thus eliminating the use of other types of binders, such as latex or resin. The process is relatively simple, and the methods of thermobonding are varied to meet the requirements for the end product. Extrusion bonding involves the extrusion of the substrate **34** onto the textile material **32**. The substrate **34** is extruded through a die at an elevated temperature to form the final structure which is then cooled.

In another aspect of the present invention, the female fastening portion **30** is applied to a disposable absorbent article. Figure 4 shows preferred embodiment of a disposable absorbent article, namely a diaper **50**, embodying the present invention. Examples of the kinds of diapers **50** to which the present invention is very readily adapted are shown in US 3,860,003 and US 4,834,735. It will be apparent from the following description that the refastenable mechanical fastening device **54** illustrated and described herein may be applied to the body portion **52** of such diapers **50**. On the other hand, it will be understood that the invention is not limited to any specific diaper construction or configuration.

Figure 4 is a partially cut-away perspective view of a diaper **50** embodying the present invention prior to it being placed on the wearer. As is visible from Figure 4, a preferred diaper **50** comprises a body portion **52** and a refastenable mechanical fastening device **54**. A preferred body portion **52** comprises a liquid pervious topsheet **56**, an absorbent core **58**, a liquid impervious backsheet **60**, and elastically contractible leg cuffs **62**; each leg cuff **62** preferably comprising a side flap **64** and one or more elastic members **66**. For simplicity purposes, only one elastic member **66** is shown in the side flap **64**. While the topsheet **56**, the absorbent core **58**, the backsheet **60**, the side flaps **64**, and the elastic members **66** may be assembled in a variety of well-known configurations, a preferred disposable diaper configuration is shown and generally described in US 3,860,003. In this preferred diaper configuration, the backsheet **60** is joined to the topsheet **56**; the absorbent core **58** is positioned between the topsheet **56** and the backsheet **60**; the side flaps **64** extend outwardly from and along each side edge of the absorbent core **58**; and the elastic member **66** is operatively associated with each side flap **64**.

Figure 4 shows the body portion **52** in which the topsheet **56** and the backsheet **60** are coextensive and have length and width dimensions generally larger than those of the absorbent core **58**. The topsheet **56** is superposed on the backsheet **60** thereby forming the periphery **68** of the body portion **52**. The periphery **68** defines the outer perimeter or in other words the outer extent of the body portion **52**. The periphery comprises the longitudinal side edges **70** and lateral end edges **72**. In the longitudinal direction, the diaper **50** has a first end region **78** and a second end region **80**.

The body portion **52** has an inside surface **74** and an outside surface **76**. In general, the outside surface **76** of the diaper **50** extends from one edge **72** to the other end edge **72** of the diaper **50** and from one longitudinal side edge **70** to the other longitudinal side edge **70** of the diaper **50**. When a backsheet **60** is used, it typically forms the outside surface **76** of the body portion **52**. The inside surface **74** is that surface of the diaper **50** opposite the outside surface **76** and in the embodiment shown is typically formed by the topsheet **56**. In general, the inside surface **74** of the diaper **50** is that surface coextensive with the outside surface **76** and which is for the greater part in contact with the wearer when the diaper **50** is worn.

The diaper **50** has a first end region **78** and a second end region **80** extending from the lateral end edges **72** of the diaper periphery **68** towards the lateral centreline of the diaper **50**. Both the first end region **78** and the second end region **80** extend a distance of about one-half the length of the diaper **50** such that the end regions **78**, **80** comprise each half of the diaper **50**. Both the first end region **78** and the second end region **80** have panels **82**. The panels **82** are those portions of the first end region **78** and the second end region **80** which overlap when the diaper **50** is fastened about the waist of the wearer. The extent to which the end regions **78** overlap and thus the extent to which the panels **82** are formed, will depend on the overall dimensions and shape of the diaper **50** and the size of the wearer.

The absorbent core **58** of the body portion **52** may be any absorbent means which is generally compressible, conformable, non-irritating to the skin of the wearer, and capable of absorbing and retaining liquids such as urine and other certain bodily discharges. The absorbent core **58** may be manufactured in a variety of sizes and shapes (for example, rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core **58** may also be varied (for example, the absorbent core **58** may have varying caliper zones, hydrophilic gradients, superabsorbent gradients, or lower average density and lower average basis weight acquisition zones; or may comprise one or more layers or structures). The total absorbent capacity of the absorbent core **58** should, however, be compatible with the design loading and the intended use of the pull-on diaper. Further, the size and absorbent capacity of the absorbent core **58** may be varied to accommodate wearers ranging from infants to adults.

The backsheet **60** is impervious to liquids (for example, urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet **60** prevents the exudates absorbed and contained in the absorbent core **58** from soiling articles which are in contact with the diaper **50** such as undergarments and bedding. The backsheet **60** may thus comprise polymeric films such as thermoplastic films of polyethylene or polypropylene, or composite materials such as film-coated non-woven material. For economic, aesthetic and ecological reasons, the backsheet **60** has an average nominal caliper, i.e., calculated caliper, of less than about 0.051 millimetres, more preferably a calculated caliper of from 0.020 millimetres to 0.036 millimetres. Preferably, the backsheet **60** is a flexible polyethylene film. As used herein, the term "polyethylene" film refers to films which are essentially made of polyethylene, however, it is understood that polyethylene film will contain a variety of additives to provide characteristics like opacity, strength requirements, colour, or any other desired characteristic that can be achieved through adding minor amounts of other substances than polyethylene into the films. The total amount of additives should be less than 45 percent, preferably less than 15 percent, by weight of film materials. Particularly, for opacity of the film, titanium dioxide is commonly used in a range of 2 to 6 percent, preferably 3.5 to 4.8 percent, by weight of the film. Exemplary films are manufactured by Tredegar Industries, Inc. of Terre Haute, Ind., USA or BP-Chemical PlasTec Rotbuchenstrasse 1, D-8000 München, Germany.

The backsheet **60** is preferably textured to provide a more clothlike appearance. Further, the backsheet **60** may also permit vapours to escape from the absorbent core **58** while still preventing exudates from passing through the backsheet **60** by, for example, being supplied with microapertures. The size of the backsheet **60** is dictated by the size of the absorbent core **58** and the exact diaper design selected.

The topsheet **56** of the body portion **52** of the present invention is compliant, soft feeling and non-irritating to the skin of the wearer. Further, the topsheet **56** is liquid pervious permitting liquids (for example, urine) to readily penetrate through its thickness. A suitable topsheet **56** may be manufactured from a wide range of materials, such as porous foams, reticulated foams, apertured films; or woven or non-woven webs of natural fibres (for example, wood or cotton fibres) or from a combination of natural and synthetic fibres. Preferably, it is made of a material that isolates the skin of the wearer from liquids retained in the absorbent core **58**.

There are a number of manufacturing techniques which may be used to manufacture the topsheet **56**. For example, the topsheet **56** may be a non-woven web of fibres. When the topsheet **56** comprises a non-woven web, the web may be spunbonded, carded, wet-laid, melt-blown, hydroentangled, hydroformed, combinations of the above, or the like. An exemplary topsheet **56** is carded and thermally bonded by means well-known to those skilled in the art of textile materials and comprise staple length polypropylene fibres having a denier of about 2.2 and has a basis weight from about 15 to about 30 grammes per square metre. As used herein, the term "staple length fibres" refer to those fibres having a length of at least 16 millimetres. A suitable topsheet **56** is manufactured by, for example, Veratec Inc., a division of International Paper Company, of Walpole, Mass., USA. A topsheet **56** particularly preferred for incontinence garments comprises a formed thermoplastic film.

The female fastening portion **30** of the fastening device **54** provides a means for securing itself and the male fastening portion **84** together to provide a secure side closure and to maintain the first end region **78** and the second end region **80** in an overlapping configuration. The female fastening portion **30** may be disposed anywhere on the diaper **50** so long as it engages the male fastening portion **84** so as to provide the side closure. For example, the female fastening portion **30** may be disposed on the outside surface **76** in the second end region **80**, on the inside surface **74** in the first end region **78**, or on any other portion of the diaper **50** which is disposed to engage the male fastening portion **84**. In addition, the female fastening portion **30** may either be a discrete separate element affixed to an element of the diaper **50** such as the topsheet **56** or the backsheet **60**. While the female fastening portion **30** can assume varying sizes and shapes and can be provided in smaller, more economical and ecological sizes depending on the use for which the disposable absorbent article is designed, it preferably comprises one or more (at least one) separate patches of material that may be larger in area than the hook fastening material **88** and which are secured to the body portion **52** to allow for a maximum fit adjustment at the waist of the wearer.

As illustrated in Figure 4, the diaper **50** has a rectangular-shaped female fastening portion **30** secured to the substrate **34**, the substrate **34** either being a film adjacent to the outside surface **76** of the body portion **52** or alternatively, the substrate **34** being simply the outside surface **76** of the body portion **52**. For the first option comprising the film, a layer of adhesive is interposed between the film and the outside surface **76** of the body portion **52**. The adhesive may comprise any suitable adhesive such as a hot melt, or the like. By way of illustration, suitable adhesives are available from Findley Adhesives, Inc. of Wauwatosa, Wis., USA under the trade designations TEF 195 and TEF 688.

The refastenable mechanical fastening device **54** comprises a male fastening portion **84**, which comprises a hook fastening material **88**. As used herein, the term "hook fastening material" is used to designate a material having engaging elements. It should be understood that the use of the term hook should be non-limiting in the sense that the engaging elements may comprise any shapes as are known in the art as long as they are adapted to engage a complementary female fastening portion **30**. As is visible from Figure 4, each male fastening portion **84** provides a fastening means for engaging the female fastening portion **30** so as to provide a secure side closure for the diaper **50**. Thus, the male fastening portion **84** comprises at least the hook fastening material **88**. Each male fastening portion **84** also preferably comprises a means for positioning the hook fastening material **88** adjacent to the female fastening portion **30** so as to achieve a side closure. Thus, the male fastening portion **84** may comprise any of the well-known configurations and securement means for achieving a side closure on a diaper **50** such as an inner fastening member secured to the inside surface **74** and/or the outside surface **76** of the body portion **52**, tape tabs, or belts. An exemplary embodiment of an inner fastening member is described in US 4,699,622. An embodiment of an incontinence undergarment using a belt suspension system is disclosed in US 4,315,508.

Each male fastening portion **84** most preferably comprises a tape tab **86**. Any of the well-known configuration and constructions of a tape tab 86 may be used. The tape tab **86** is provided on both longitudinal side edges **70** of the body portion **52**, most preferably in the first end region **78**. A particularly preferred tape tab **86** has a tape tab fastening surface **90** and a tab backing surface **92**. The tape tab fastening surface **90** is the surface of the tape tab **86** designed to engage the female fastening portion **30** of the present invention. Thus, the tape tab fastening surface **90** of the tape tab **86** will generally correspond to the inside surface **74** of the body portion **52**. The tape tab backing surface **92** is that surface opposite to the tape tab fastening surface **90** and generally corresponds to the outside surface **76** of the body portion **52**. The tape tab backing surface **92** is thus generally exposed during wear of the diaper **50**.

The hook fastening material **88** is preferably joined to either the body portion **52** or the tape tab **86**. As used herein, the term "joined" encompasses configurations whereby the hook fastening material **88** is releasably secured to the diaper **50** so the hook fastening material **88** may be removed from the diaper **50** or its location during use and whereby the hook fastening material **88** is securely fastened to the diaper **50**. Joined is also used to denote that the hook fastening material **88** may be directly joined to the diaper **50** or may be indirectly joined to the diaper **50** such as by releasably securing or affixing the hook fastening material **88** to an intermediate member which, in turn, is releasably secured or affixed to the diaper **50**. Preferably, as shown in Figure 4, the hook fastening material **88** is directly affixed to the connective portion **102** of the tape tab **86** by a second tab attachment means **112**.

In addition, the hook fastening material **88** may be positioned anywhere on the diaper **50**. When the male fastening portion **84** comprises an inner fastening member, the hook fastening material **88** is preferably positioned in the panels **82** of the first end region **78** adjacent to the longitudinal side edges **70**. When the male fastening portion **84** comprises a tape tab **86**, the hook fastening material **88** is preferably positioned either on all of or at least a portion of the tape tab fastening surface **90**, or preferably on all of or at least a portion of the connective portion **102**. Most preferably, the hook fastening material **88** is disposed on the connective portion **102** on the tape tab fastening surface **90**.

In order to measure the shear stresses and peel forces of the female fastening portion **30** and the male fastening portion **84** of the refastenable mechanical fastening device **54**, reference is made to a suitable test method, which is described below in the section entitled "Description of test procedure". The values measured for a selection of samples (i.e., for the female fastening portion **30** when engaged with the male fastening portion **84**) were reported to be greater than 500 grammes for the shear stresses and to be at least 85 newtons per metre for the peel force with preferably 4 sigma < |average measurement - 85 newtons per metre|.

In a further embodiment of the present invention, where the substrate **34** comprises a film adjacent to the outside surface **76** of the body portion **52** as described hereinabove, the film is printed. For the alternative embodiment of the present invention where the substrate **34** comprises the outside surface **76** of the body portion **52**, the outside surface **76** of the body portion **52** may be printed. Printing is done by means known to the man skilled in the art.

In use, the diaper **50 i**s applied to the wearer by positioning the first end region **78** under the back of the wearer and drawing the remainder of the diaper **50** between the legs of the wearer so the second end region **80** is positioned across the front of the wearer. The connective portion **102** of the tape tabs **86** are then positioned adjacent to the female fastening portion **30** positioned on the outer surface **76** of the second end region **80** so the hook fastening material **88** which is disposed on the tape tab fastening surface **90** will respectively engage the textile material **32** of the female fastening portion **30** to form a side closure.

### Description of test procedure

For the purposes of the present invention, a method is herein described which describes the procedure for measuring the shear stresses (using a 500 gramme weight) and peel force of the female fastening portion **30** and male fastening portion **84** for a refastenable mechanical fastening device **54** according to the teachings of the present invention. The testing method is applicable to a female fastening portion **30** in the form of a loop raw material and a male fastening portion **84** in the form of a hook raw material.

### Apparatus required

- Conditioned room: Controlled to 22 degrees celsius ± 2 degrees celsius, 50 percent ± 2 percent relative humidity.
- Scissors: Usual pair.
- Test stand: Clamps designed to hold the 500 gramme weight and mounted in such a way so that the sample can hang free.
- Paper strips: Cut width of 2.54 centimetres from copying paper 80 grammes per square metre SB or equivalent.
- Loop material: As described above.
- Hook material: Normal production material specification 85379.
- Inch cutter: Supplied from Thwing Albert Instrument Company, Philadelphia, USA.
- 500 gramme weight: Usual type with clamp attached.
- Electronic tensile tester: Supplied from Zwick 1445, Zwick GmbH & Co., 7900 Ulm with a 100 newton load cell or equivalent.

### Sample preparation

Condition the test material at 22 degrees celsius and 50 percent relative humidity for a minimum of two hours prior to testing. Prepare a hook sample strip for each loop sample strip to be tested.

### Loop strip preparation:-

With an inch cutter, cut a strip (2.54 centimetres machine direction by cross direction width) out of the loop material. Cut strips in half. Be aware of material orientation - loops up and loops down.

### Hook material preparation:-

Remove the outer layer of the sample roll to ensure no contaminated or damaged hook material is measured. Ensure that the hooks unwind from the right side. Tape the hooks onto a 2.54 centimetre wide and at least 100 millimetre long paper strip.

### Shear test

- Take the loop strip and insert in the clip of the test stand.
- Take the hook strip and clamp it into the 500 gramme weight clip.
- Take the hook strip with the weight and press it into the loop strip using the thumb and forefinger.
- Release it carefully and let it hang for 5 seconds.
- Remove the weight from the hook strip.
- Remove the sheared sample from the test stand and perform the peel test on the tensile tester.

### Instrument preparation

Before beginning the peel test, the tensile tester needs to be calibrated. Reference should be made to the manufacturer's instruction manual. The parameters should be set as follows:
- Gauge length: = 50 millimetres (0.2 inches)
- Pre-force: = 0.2 newtons
- Crosshead speed: = 305 millimetres per minute (12 inches per minute)
- Measuring distance: = 40 millimetres

### Peel test

- Place the hook strip into the upper (or moving) jaw of the tensile tester.
- Place the loop strip in the lower (or static) jaw.
- Start the crosshead to initiate the test.
- Repeat the procedure for both direction configurations of closure.

### Reporting of data

- Report each sample that fails the shear test.
- Report the peak force (newton) and average force (newton) for the peel test. Samples which failed the shear test will be reported as zero.
- Calculate the total average, standard deviation (maximum and minimum) and the average, standard deviation (maximum and minimum) for each orientation separately.
- Print the graphs of the peel force curves.

### GLOSSARY

- 30: Female fastening portion
- 32: Textile material
- 34: Substrate
- 38: Loops
- 40: Courses
- 42: Wales
- 50: Diaper
- 52: Body portion
- 54: Mechanical fastening device
- 56: Topsheet
- 58: Absorbent core
- 60: Backsheet
- 62: Leg cuffs
- 64: Side flaps
- 66: Elastic members
- 68: Periphery of body portion
- 70: Longitudinal side edges
- 72: Lateral end edges
- 74: Inside surface of body portion
- 76: Outside surface of body portion
- 78: First end region
- 80: Second end region
- 82: Panels
- 84: Male fastening portion
- 86: Tape tab
- 88: Hook fastening material
- 90: Tape tab fastening surface
- 92: Tape tab backing surface
- 102: Connective portion
- 112: Second tab attachment means
- C: Direction for equivalent distribution of forces
- D: Direction for equivalent distribution of forces

## Claims

1. Female fastening portion (30) for a refastenable mechanical fastening device (54), said female fastening portion (30) being capable of engaging a complementary male fastening portion (84), said female fastening portion (30) comprising a textile material (32), said textile material (32) being disposed adjacent a substrate (34), whereby said textile material (32) is non-adhesively bonded to said substrate (34),
characterised in that
said textile material (32) has a basis weight not greater than 60 grammes per square metre.

2. Female fastening portion (30) according to claim 1 wherein said textile material (32) comprises a basis weight not greater than 40 grammes per square metre, preferably not greater than 34 grammes per square metre and more preferably, not greater than 28 grammes per square metre.

3. Female fastening portion (30) according to any of the preceding claims wherein said textile material (32) is thermobonded to said substrate (34).

4. Female fastening portion (30) according to claims 1 and 2 wherein said textile material (32) is extrusion bonded to said substrate (34).

5. Female fastening portion (30) according to any of the preceding claims wherein said textile material (32) comprises a three bar warp pattern comprising loops (38), courses (40) and wales (42).

6. Female fastening portion (30) according to claims 1 to 4 wherein said textile material (32) has a two bar warp pattern comprising courses (40) and wales (42).

7. Female fastening portion (30) according to claim 1 wherein said female fastening portion (30) when engaged with said male fastening portion (84) has a peel force resistance value of at least 85 newtons per metre.

8. Disposable absorbent article (50) comprising a body portion (52) having an inside surface (74), an outside surface (76), and said female fastening portion (30) according to claims 1 to 7 wherein said substrate (34) comprises a film adjacent said outside surface (76) of said body portion (52) with a layer of adhesive being interposed between said film and said outside surface (76) of said body portion (52).

9. Disposable absorbent article (50) according to claim 8 wherein said film is printed.

10. Disposable absorbent article (50) comprising a body portion (52) having an inside surface (74), an outside surface (76), and said female fastening portion (30) according to claims 1 to 7 wherein said substrate (34) comprises said outside surface (76) of said body portion (52).

11. Disposable absorbent article (50) according to claim 10 wherein said outside surface (76) of said body portion (52) is printed.
